# EUROPEAN PATENT SPECIFICATION

(11) **EP 3 435 085 B1**
(45) Date of publication and mention of the grant of the patent: **24.07.2019**
(21) Application number: 18152026.3
(22) Date of filing: 17.01.2018
(51) Int. Cl.: G01N 33/68

(54) **USE OF COMPOSITION AND KIT FOR DIAGNOSING BEHAVIORAL ADDICTION, AND METHOD OF DETECTING PRESENILIN-1 FOR DIAGNOSIS OF BEHAVIORAL ADDICTION.**
VERWENDUG EINER ZUSAMMENSETZUNG UND VERWENDUG EINES KITS ZUR DIAGNOSE VON VERHALTENSSUCHT UND VERFAHREN ZUR ERKENNUNG VON PRESENILIN-1 ZUR DIAGNOSE VON VERHALTENSSUCHT.
UTILISATION DE COMPOSITION ET TROUSSE PERMETTANT DE DIAGNOSTIQUER L'ACCOUTUMANCE COMPORTEMENTALE ET PROCÉDÉ DE DÉTECTION DE PRÉSÉNILINE-1 POUR LE DIAGNOSTIC DE L'ACCOUTUMANCE COMPORTEMENTALE.

(30) Priority: 26.07.2017 KR 20170094978
(43) Date of publication of application: 30.01.2019
(73) Proprietor: Korea Institute of Science and Technology, Seoul 02792 (KR)
(72) Inventor: LEE, Ji Eun, 02792 Seoul (KR); CHEON, Dong Huey, 02792 Seoul (KR); PARK, Yae Eun, 02792 Seoul (KR); LEE, Jung Eun, 02792 Seoul (KR); LEE, Cheol Ju, 02792 Seoul (KR); KIM, Dai Jin, 06591 Seoul (KR); CHUN, Ji Won, 06591 Seoul (KR); CHOI, Jung Seok, 07061 Seoul (KR)
(74) Representative: Gill, Siân Victoria

(56) References cited:
- CN-A- 104 711 334
- CN-A- 105 969 885
- Raybio Human Presenilin ET AL: "RayBio Human Presenilin 1 ELISA Kit", , 2 June 2016 (2016-06-02), XP055463177, Retrieved from the Internet: URL:https://www.raybiotech.com/files/manua l/ELISA/ELH-PSEN1.pdf [retrieved on 2018-03-27]
- TRIFILIEFF PIERRE ET AL: "Blunted Dopamine Transmission in Addiction: Potential Mechanisms and Implications for Behavior", SEMINARS IN NUCLEAR MEDICINE, ELSEVIER, AMSTERDAM, NL, vol. 47, no. 1, 13 October 2016 (2016-10-13), pages 64-74, XP029848086, ISSN: 0001-2998, DOI: 10.1053/J.SEMNUCLMED.2016.09.003
- YAU YVONNE H C ET AL: "Gambling disorder and other behavioural addictions: recognition and treatment", HARVARD REVIEW OF PSYCHIATRY,, vol. 23, no. 2, 1 March 2015 (2015-03-01), pages 134-146, XP009195470, ISSN: 1465-7309, DOI: 10.1097/HRP.0000000000000051

## Description

### BACKGROUND

### 1. Field

One or more embodiments relate to a composition and a kit for use in diagnosing behavioral addictions, and a method of detecting presenilin-1 for diagnosis of behavioral addictions using the same.

### 2. Description of the Related Art

Behavioral addictions are addictions to any behavior, process, or activity, such as gambling addiction, sex addiction, pathological use of Internet or game, etc. Brain circuits are involved in behavioral addictions, and resistance and withdrawal may occur. Impulsivity dominates at the early stages of development, and compulsivity is the key to maintain behavior. In modern society, Internet addiction, game addiction, and obsessive disorder, together with alcoholism and drug addiction, are becoming more prominent.

Biomarkers for psychiatric symptoms, for example, autism, Parkinson's disease, dementia, attention deficit hyperactivity disorder (ADHD), alcoholism, drug addiction, obsessive-compulsive disorder, etc., are known (US Patent Publication NO. 2005/0084880A1 (2005.04.21), Korean Patent NO. 10-1157526 (2016.06.27), etc.). However, for diagnosis of behavioral addiction, in particular, Internet addiction or game addiction, brain imaging through magnetic resonance imaging (MRI) or self-assessment through a questionnaire survey is mainly used, but there are no effective biomarkers for diagnosis thereof.

The User Manual for RayBio® Human Presenilin 1 ELISA Kit (revised on June 2, 2016) states that the kit is an in vitro enzyme-linked immunosorbent assay for the quantitative measurement of human Presenilin 1 in serum, plasma and cell culture supernatants, employing an antibody specific for human Presenilin 1 coated on a 96-well plate. CN 105969885A discloses a kit to detect genes such as presenilin 1 and this kit may be used to diagnose Alzheimer's disease. CN 104711334A discloses an in-situ hybridization kit to detect presenilin 1 mRNA for diagnosing early stage Alzheimer's disease. Trifilieff, P., et al, Seminars in Nuclear Medicine, vol. 47, no. 1, 13 October 2016, pages 64-74 disclose that Positron Emission Tomography (PET) imaging consistently shows blunted striatal dopamine release and decreased dopamine D2 receptor availability in addiction. The data suggest that these are biomarkers both for the development of addiction and resistance to treatment. Yau et al, Harvard Review of Psychiatry, vol. 23, no. 2, 1 March 2015, pages 134-146 disclose that certain non-substance behaviors, such as gambling, internet use, video-game playing, sex, eating and shopping, bear resemblance to alcohol and drug dependence.

Accordingly, there is a need for the development of biomarkers capable of diagnosing behavioral addictions easily and inexpensively with high accuracy and specificity, not an expensive and inconvenient brain imaging diagnosis or a self-diagnosis method based on subjective assessment.

### SUMMARY

One or more embodiments include use of a composition for diagnosing behavioral addictions.

One or more embodiments include use of a kit for diagnosing behavioral addictions.

One or more embodiments include a method of detecting presenilin-1 for the diagnosis of behavioral addictions.

### BRIEF DESCRIPTION OF THE DRAWINGS

These and/or other aspects will become apparent and more readily appreciated from the following description of the embodiments, taken in conjunction with the accompanying drawings in which:
FIG. 1 is a graph showing results of high-performance liquid chromatography in which 14 high-abundance proteins were removed from a serum sample using immunoaffinity column (x axis: time (min), y axis: milli Absorbance Unit (mAU));
FIG. 2A is an SDS-PAGE gel image before and after depletion of the high-abundance proteins from four serum samples of normal control group, and FIG. 2B is an SDS-PAGE gel image before and after depletion of the high-abundance proteins from four serum samples of Internet or game addiction group (left: before depletion of the high-abundance proteins from four individual serum samples, right: after depletion of the high-abundance proteins from four individual serum samples, M: molecular weight marker (kDa), 1 to 4: Lanes 1 to 4);
FIG. 3A is an image obtained by performing an antibody array of 493 kinds of proteins, FIG. 3B is an image showing a comparison of antibody array spots of presenilin-1 between the control group and the Internet or game addiction group, and FIG. 3C is a bar graph showing relative intensities of antibody array spots of presenilin-1; and
FIG. 4 is a graph showing results obtained by analyzing band intensities of presenilin-1 from immunoblots of a normal control group (control group) and an addiction group including Internet or game addicts (addiction group).

### DETAILED DESCRIPTION

According to one or more embodiments, the use of a composition in diagnosing behavioral addictions is provided, wherein the composition includes an agent capable of specifically binding to presenilin-1 protein or a polynucleotide encoding presenilin-1 protein in order to measure an expression level of presenilin-1 protein in a biological sample from a subject suspected of having a behavioral addiction, wherein the agent is an antibody or an antigen-binding fragment thereof specifically binding to presenilin-1 protein; or a nucleic acid comprising a polynucleotide which is identical or complementary to a polynucleotide encoding presenilin-1 protein or a fragment thereof. This allows measuring of an expression level of Presenilin-1 protein or a fragment thereof.

The term "behavioral addiction" refers to an addiction to any behavior, process, or activity. Behavioral addictions may involve resistance and withdrawal. The behavioral addictions may be selected from the group consisting of Internet addiction, smartphone addiction, game addiction, gambling addiction, religious addiction, sex addiction, shopping addiction, exercise addition, and work addiction. Internet addiction refers to an addiction state which is caused by pathological or compulsive Internet use to affect physical, mental, or social life. Game addiction refers to an addiction state which is caused by pathological or compulsive game immersion to affect physical, mental, or social life. The Internet or game includes those by use of all mediums such as computers, mobile phones, smartphones, TVs, etc.

Presenilin-1 (PSN-1) is one of four key proteins of a gamma secretase complex, and may be also called S182 protein. The gamma secretase complex is known to play a role in the formation of β-amyloid from an amyloid precursor protein. Considering that accumulation of β-amyloid is a major pathogenesis of Alzheimer's disease, PSN-1 is presumed to be associated with Alzheimer's disease. In addition, PSN-1 is also known to be important in cancer. PSN-1 may be a protein that is expressed by a PSEN1 gene. PSN-1 may be cleaved into a PSN-1 NTF subunit, a PSN-1 CTF subunit, or a PSN-1 CTF12 subunit. PSN-1 may include an amino acid sequence of Uniprot ID NO: P49768 in human.

The fragment may be an immunogenic polypeptide as a part of PSN-1 protein.

The agent may be an antibody or an antigen-binding fragment thereof specifically binding to PSN-1 protein or a fragment thereof. The antibody may be a polyclonal antibody or a monoclonal antibody. The term "antibody" may be used interchangeably with "immunoglobulin". The antibody may be a polyclonal antibody or a monoclonal antibody. The antibody may be a full-length antibody. The antigen-binding fragment refers to a polypeptide including an antigen-binding site. The antigen-binding fragment may be a single-domain antibody, Fab, Fab', or scFv. The antibody or antigen-binding fragment may be attached to a solid support. The solid support may be, for example, a surface of a metal chip, plate or well. The antibody or antigen-binding fragment may be an anti-PSN-1 antibody or an antigen-binding fragment thereof.

The agent may be a nucleic acid including a polynucleotide which is identical or complementary to a polynucleotide encoding PSN-1 protein or a fragment thereof. The nucleic acid may be a primer or a probe. The primer or the probe may be labeled with a fluorescent material, a chemiluminescent material, a radioactive isotope, etc. at the terminus or internally.

According to one or more embodiments, use of a kit in diagnosing behavioral addictions is provided, wherein the kit includes an agent capable of specifically binding to presenilin-1 protein or a polynucleotide encoding presenilin-1 protein in order to measure an expression level of presenilin-1 protein in a biological sample from a subject suspected of having a behavioral addiction, wherein the agent is an antibody or an antigen-binding fragment thereof specifically binding to presenilin-1 protein; or a nucleic acid comprising a polynucleotide which is identical or complementary to a polynucleotide encoding presenilin-1 protein or a fragment thereof. This allows measuring of an expression level of PSN-1 protein or a fragment thereof.

The kit may further include a sample required for the diagnosis of behavioral addictions. For immunological detection of the solid support, antibody, or antigen-binding fragment, the kit may include a substrate, an appropriate buffer solution, a color development enzyme, a fluorescent-labeled secondary antibody, or a color development substrate. For nucleotide detection, the kit may include a polymerase, a buffer, a nucleotide, a coenzyme, a fluorescent material, or a combination thereof. The polymerase may be, for example, Taq polymerase.

According to one or more embodiments, a method of detecting PSN-1 for the diagnosis of behavioral addictions includes: measuring an expression level of PSN-1 protein or a fragment thereof in a biological sample which is separated from a subject suspected of having a behavioral addiction; and comparing the measured expression level of the protein of a normal control group.

The method includes measuring an expression level of PSN-1 protein or a fragment thereof in a biological sample which is separated from a subject suspected of having a behavioral addiction.

The subject may be a mammal, for example, a human, a cow, a horse, a pig, a dog, a sheep, a goat, or a cat. The subject may be a subject suffering from a behavioral addiction or a subject suspected of having a behavioral addiction.

The biological sample refers to a sample obtained from the subject. The biological sample may be, for example, blood, plasma, serum, bone marrow fluid, lymphatic fluid, saliva, lachrymal fluid, mucosal fluid, amniotic fluid, or a combination thereof.

The measuring of the expression level may include incubating the biological sample with an antibody specifically binding to PSN-1 protein or a fragment thereof.

The measuring of the expression level may be performed by electrophoresis, immunoblotting, enzyme-linked immunosorbent assay (ELISA), protein chips, immunoprecipitation, microarray, northern blotting, polymerase chain reaction (PCR), or a combination thereof. The electrophoresis may be SDS-PAGE, isoelectric focusing, two-dimensional electrophoresis, or a combination thereof. The PCR may be real-time PCR or reverse transcription PCR.

The method includes comparing the measured expression level with a protein expression level of a normal control group.

In the method, a quantity of the measured expression level of PSN-1 may be increased, compared with an expression level of PSN-1 derived from a sample of a normal control group. The normal control group means a negative control group having no behavioral addiction. For example, when the protein level of the detected PSN-1 protein is greater than that of the PSN-1 protein of the normal control group, the subject may be diagnosed as having or at high risk of having a behavioral addiction, for example, Internet addiction or game addiction. The subject diagnosed as having a behavioral addiction, for example, Internet addiction or game addiction may be subjected to psychosocial therapy or drug treatment. The drug may be, for example, naltrexone, disulfiram, methadone, chlordiazepoxide, acamprosate, bupropion, varenicline, buprenorphine, or a combination thereof.

Reference will now be made in detail to embodiments, examples of which are illustrated in the accompanying drawings, wherein like reference numerals refer to like elements throughout. In this regard, the present embodiments may have different forms and should not be construed as being limited to the descriptions set forth herein. Accordingly, the embodiments are merely described below, by referring to the figures, to explain aspects of the present description.

Hereinafter, the present invention will be described in more detail with reference to Examples. However, these Examples are for illustrative purposes only, and the scope of the present invention is not intended to be limited by these Examples.

### Example 1. Identification of differentially expressed proteins among serum proteins of Internet or game addiction group

### 1. Preparation of samples of Internet or game addiction groups

Serum samples of Internet or game addiction patients (n=4) were collected from Seoul St. Mary's Hospital, Catholic University of South Korea. Serum samples of normal persons (n=4) who were not diagnosed as Internet or game addiction were collected as a negative control.

To prevent protein degradation of the serum samples, a complete Mini EDTA-free protease inhibitor cocktail (Roche) was added to the collected serum immediately after collecting the serum. One tablet of the inhibitor was dissolved in 200 µl of a phosphate buffered saline (PBS) to prepare a 50 X stock according to the manufacturer's protocol, and then added to each serum sample at a final concentration of 1.5 X. The inhibitor-added serum samples were quantified and stored at -80°C before use in experiments.

### 2. Depletion of high-abundance proteins in serum samples

To remove abundant proteins in the serum, such as albumin or immunoglobulin G, the collected serum samples were depleted.

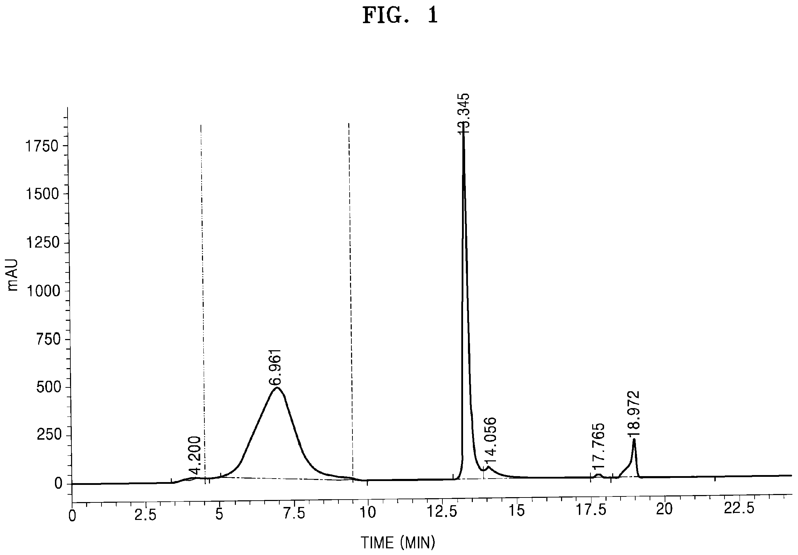

In detail, the serum samples collected in 1. were prepared at a concentration of 54.8 µg/µl which is a minimum concentration of a serum sample among 8 serum samples, respectively, and experiments were performed under the same conditions. The prepared serum samples were diluted 6 times with a buffer solution provided by the manufacturer, and each of the diluted samples was filtered using a 0.22 µm spin filter. 100 µl of the filtered sample was injected into a Multiple Affinity Removal System (MARS) 14 column (Agilent), and the top 14 high-abundance proteins (albumin, immunoglobulin G, alpha-1-antitrypsin, immunoglobulin A, transferrin, haptoglobin, fibrinogen, alpha-2-macroglobulin, alpha-1-acid glycoprotein, immunoglobulin M, apolipoprotein A-I, apolipoprotein A-II, complement C3, and transthyretin) were removed from each sample was removed using an Agilent 1100 HPLC system. Results of measuring absorbance of the serum sample depleted of the top 14 high-abundance proteins by the MARS 14 column in real time are shown in FIG. 1 (-••-••-: a starting point for collecting the depleted serum, - - - - : an end point for collecting the depleted serum).

### 3. Concentration and quantification of depleted serum sample

A 3K centrifugal filter (Amicon Ultra, Millipore) was conditioned by adding 400 µl of water (HPLC grade, J.T Baker) twice and 400 µl of 10 mM HEPES buffer (pH 8.0) twice thereto.

Each of the serum samples depleted in 2. was diluted 5 times with 10 mM HEPES buffer. Each diluted sample was applied to the conditioned filter, and centrifuged at a speed of 14,000x g and 10°C for 10 minutes to concentrate the sample. Thereafter, the sample-containing filter was washed with 10 mM HEPES buffer 5 times. The centrifugal filter containing the depleted serum sample was turned over and put in a new tube, and centrifuged at a speed of 3,000x g and 10°C for 2 minutes to recover the concentrated sample.

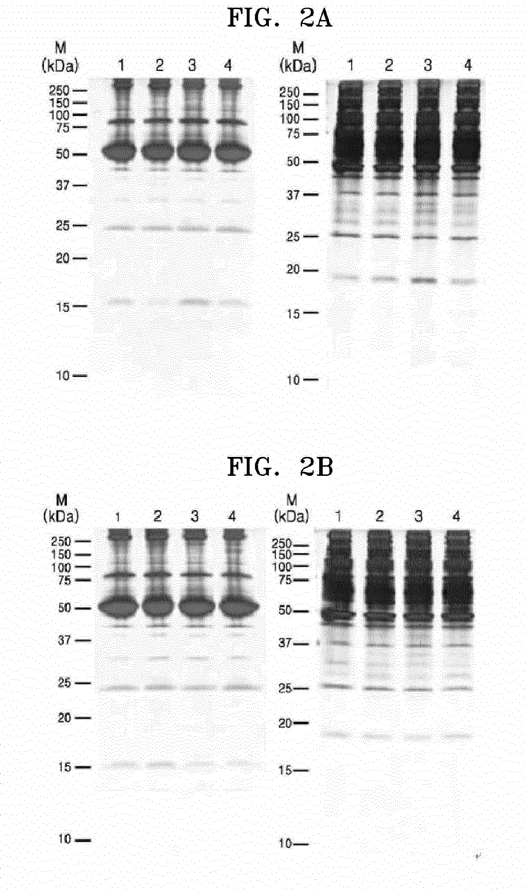

The concentrated samples were quantified by a bicinchoninic acid (BCA) assay. Equal amounts of the proteins were separated by SDS-PAGE, and the electrophoresed gels were stained by silver staining for visualization of each depleted serum sample. Images of the stained gels are shown in FIGS. 2A and 2B (FIG. 2A: SDS-PAGE gel image before and after depletion of the serum samples of the normal control group, FIG. 2B: SDS-PAGE gel image before and after depletion of the serum samples of the Internet or game addiction group).

### 4. Comparative analysis of protein expression from normal and addiction samples using antibody array

4 serum samples of the normal control group and 4 serum samples of the addiction group including Internet or game addicts were, respectively, pooled at a protein concentration of 500 µg/1.5 ml.

3.6 µl of a biotinylation reagent (RayBiotech, Inc.) was added to each of the pooled serum samples, and incubated for 30 minutes at room temperature, and then 5 µl of a stop solution (RayBiotech, Inc.) was added to terminate the reaction. The reactant was filtered through a size exclusion column (RayBiotech, Inc.) to remove remaining biotin in the sample.

The human antibody 493 arrays (membrane type, available from RayBiotech, Inc.) were blocked for 1 hour at room temperature by a blocking buffer solution. The blocking buffer solution was also added to 1.5 ml of a biotin-labeled sample to be diluted at a total volume of 14 ml. Each sample from either control or addiction group was applied to the array, and incubated at 4°C overnight.

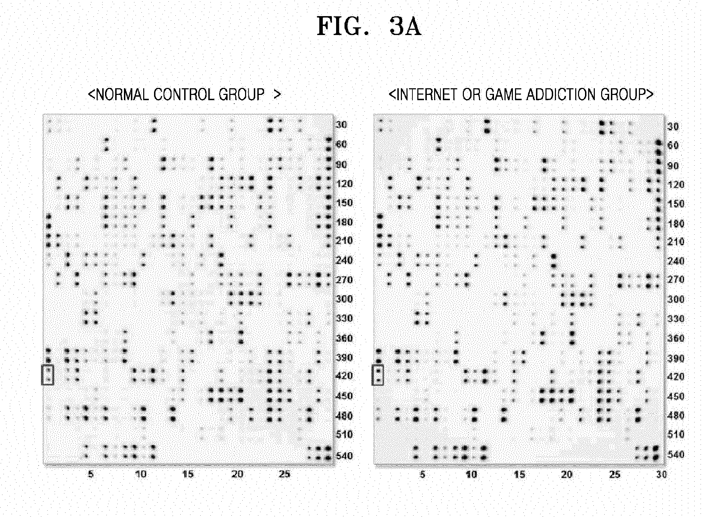

Thereafter, the array was washed with a washing buffer included in the antibody array kit, and a streptavidin-HRP conjugated solution (RayBiotech, Inc.) was added to the array, followed by incubation at room temperature for 2 hours. The array was washed, and signals from the array were detected using chemiluminescence. The results of the antibody arrays containing 493 proteins from the control and the Internet and game addiction samples are shown in FIG. 3A.

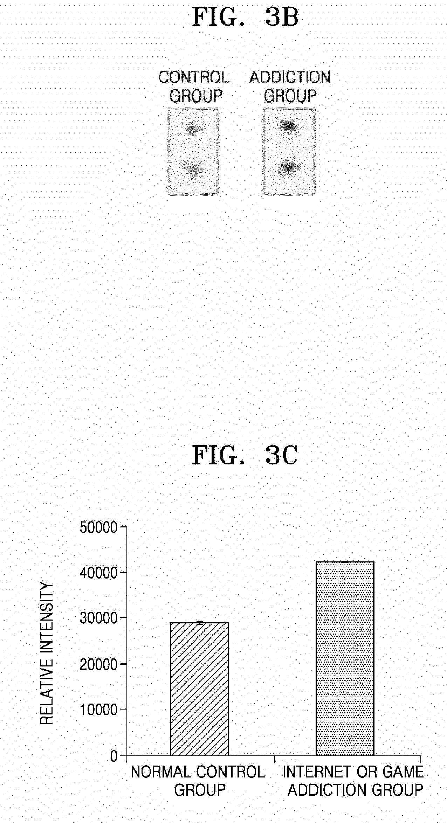

As shown in FIG. 3A, proteins, which were differentially expressed in the samples of the normal control group and the Internet or game addiction group, were identified. Among the proteins, which were differentially expressed in the samples of the normal control group and the Internet or game addiction group, PSN-1 was selected as a biomarker to differentiate the control and Internet or game addiction group, and an antibody array image of the PSN-1 protein is shown in FIG. 3B. In the antibody array image of the PSN-1 protein, relative intensities of the spots were quantified by using TotalLab 1D analysis software (Nonlinear Dynamics), and relative intensities of the spots in the control group and the Internet or game addiction group are shown in FIG. 3C.

As shown in FIG. 3C, the PSN-1 expression was significantly increased in the Internet or game addiction group, compared to the control group.

### 5. Verification of differential expression using immunoblotting

An expression pattern of the PSN-1 protein selected as a biomarker candidate in 4. was assayed. For assay, samples of 24 persons of the control group and 24 persons of the Internet or game addiction group provided by Boramae Medical Center, Seoul National University were used to perform experiments.

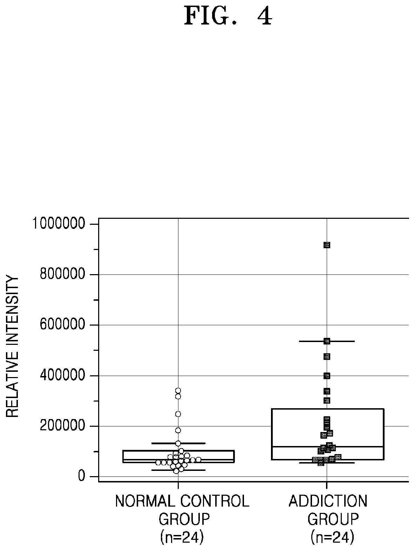

10 µg of a sample in which 14 high-abundance proteins present in the control group and the Internet or game addiction group were reduced was subjected to electrophoresis, followed by immunoblotting using anti-PSN-1 antibodies (available from Genetex) and secondary antibodies (available from Thermo Fisher Scientific). Images obtained by immunoblotting were analyzed, wherein, for normalization, the band intensity of the QC sample was used. The results analyzed by Mann-Whitney U-test are shown in FIG. 4 (addiction group: Internet or game addiction group, *P* = 0.0032).

As shown in FIG. 4, it was confirmed that the PSN-1 expression was significantly increased in the Internet or game addiction group, compared to the normal control group, and that is, the PSN-1 protein showed excellent accuracy as a biomarker for the Internet and game addictions.

As described above, a composition or a kit for diagnosing behavioral addictions and a method of detecting PSN-1 for the diagnosis of behavioral addictions using the same according to an aspect may be simply used to diagnose behavioral addictions, for example, Internet addiction or game addiction, with high accuracy and specificity.

## Claims

1. Use of a composition in the diagnosis of behavioral addictions, the composition comprising an agent capable of specifically binding to presenilin-i protein or a polynucleotide encoding presenilin-1 protein in order to measure an expression level of presenilin-1 protein in a biological sample from a subject suspected of having a behavioral addiction,
wherein the agent is an antibody or an antigen-binding fragment thereof specifically binding to presenilin-1 protein; or a nucleic acid comprising a polynucleotide which is identical or complementary to a polynucleotide encoding presenilin-1 protein or a fragment thereof.

2. The use of claim 1, wherein the behavioral addiction is selected from the group consisting of Internet addiction, game addiction, gambling addiction, sex addiction, shopping addiction, exercise addition, and work addiction.

3. The use of claim 1, wherein the antibody is a polyclonal antibody or a monoclonal antibody.

4. The use of claim 1, wherein the nucleic acid is a primer or a probe.

5. Use of a kit in the diagnosis of behavioral addictions, the kit comprising an agent capable of specifically binding to presenilin-1 protein or a polynucleotide encoding presenilin-1 protein in order to measure an expression level of presenilin-1 protein in a biological sample from a subject suspected of having a behavioral addiction,
wherein the agent is an antibody or an antigen-binding fragment thereof specifically binding to presenilin-1 protein; or a nucleic acid comprising a polynucleotide which is identical or complementary to a polynucleotide encoding presenilin-1 protein or a fragment thereof.

6. A method of detecting presenilin-1 for the diagnosis of behavioral addictions, the method comprising:
measuring an expression level of presenilin-1 protein in a biological sample which is separated from a subject suspected of having a behavioral addiction; and
comparing the measured expression level with an expression level of presenilin-1 protein of a normal control group.

7. The method of claim 6, wherein the biological sample is blood, plasma, serum, bone marrow fluid, lymphatic fluid, saliva, lachrymal fluid, mucosal fluid, amniotic fluid, or a combination thereof.

8. The method of claim 6, wherein the measuring comprises incubating the biological sample with an antibody specifically binding to presenilin-i protein.

9. The method of claim 6, wherein the measuring is performed by electrophoresis, immunoblotting, enzyme-linked immunosorbent assay (ELISA), protein chips, immunoprecipitation, microarray, northern blotting, polymerase chain reaction (PCR), or a combination thereof.

## Patentansprüche

1. Verwendung einer Zusammensetzung bei der Diagnose von Verhaltenssüchten, wobei die Zusammensetzung einen Wirkstoff umfasst, der zum spezifischen Binden an Presenilin-1-Protein oder ein Presenilin-1-Protein codierendes Polynukleotid fähig ist, um ein Expressionsniveau von Presenilin-1-Protein in einer biologischen Probe aus einem Subjekt mit Verdacht auf eine Verhaltenssucht zu messen,
wobei der Wirkstoff ein Antikörper oder ein antigenbindendes Fragment davon ist, der bzw. das spezifisch an Presenilin-1-Protein bindet; oder eine Nukleinsäure umfassend ein Polynukleotid, das identisch oder komplementär zu einem Polynukleotid, das Presenilin-1-Protein oder ein Fragment davon codiert, ist.

2. Verwendung nach Anspruch 1, wobei die Verhaltenssucht ausgewählt ist aus der Gruppe bestehend aus Internetsucht, Spielsucht, Glücksspielsucht, Sexsucht, Einkaufssucht, Übungssucht und Arbeitssucht.

3. Verwendung nach Anspruch 1, wobei der Antikörper ein polyklonaler Antikörper oder ein monoklonaler Antikörper ist.

4. Verwendung nach Anspruch 1, wobei die Nukleinsäure ein Primer oder eine Sonde ist.

5. Verwendung eines Kits bei der Diagnose von Verhaltenssüchten, wobei das Kit einen Wirkstoff umfasst, der zum spezifischen Binden an Presenilin-1-Protein oder ein Presenilin-1-Protein codierendes Polynukleotid fähig ist, um ein Expressionsniveau von Presenilin-1-Protein in einer biologischen Probe aus einem Subjekt mit Verdacht auf eine Verhaltenssucht zu messen,
wobei der Wirkstoff ein Antikörper oder ein antigenbindendes Fragment davon ist, der bzw. das spezifisch an Presenilin-1-Protein bindet; oder eine Nukleinsäure umfassend ein Polynukleotid, das identisch oder komplementär zu einem Polynukleotid, das Presenilin-1-Protein oder ein Fragment davon codiert, ist.

6. Verfahren zur Erkennung von Presenilin-1 zur Diagnose von Verhaltenssüchten, wobei das Verfahren umfasst:
Messen eines Expressionsniveaus von Presenilin-1-Protein in einer biologischen Probe, die aus einem Subjekt mit Verdacht auf eine Verhaltenssucht isoliert wird; und
Vergleichen des gemessenen Expressionsniveaus mit einem Expressionsniveau von Presenilin-1-Protein einer normalen Kontrollgruppe.

7. Verfahren nach Anspruch 6, wobei die biologische Probe Blut, Plasma, Serum, Knochenmarkflüssigkeit, Lymphflüssigkeit, Speichel, Tränenflüssigkeit, Schleimhautflüssigkeit, Fruchtwasser oder eine Kombination davon ist.

8. Verfahren nach Anspruch 6, wobei das Messen das Inkubieren der biologischen Probe mit einem Antikörper, der spezifisch an Presenilin-1-Protein bindet, umfasst.

9. Verfahren nach Anspruch 6, wobei das Messen durch Elektrophorese, Immunblotting, Enzymimmunoassay (ELISA), Proteinchips, Immunpräzipitation, Mikroarray, Northern Blotting, Polymerasekettenreaktion (PCR) oder eine Kombination davon erfolgt.

## Revendications

1. Utilisation d'une composition dans le diagnostic d'addictions comportementales, la composition comprenant un agent capable de se lier spécifiquement à la protéine préséniline-1 ou un polynucléotide codant pour la protéine préséniline-1 afin de mesurer un niveau d'expression de la protéine préséniline-1 dans un échantillon biologique d'un sujet suspecté de présenter une addiction comportementale, dans laquelle l'agent est un anticorps ou un fragment de liaison aux antigènes de celui-ci se liant spécifiquement à la protéine préséniline-1 ; ou un acide nucléique comprenant un polynucléotide qui est identique ou complémentaire à un polynucléotide codant pour la protéine préséniline-1 ou un fragment de celui-ci.

2. Utilisation selon la revendication 1, dans laquelle l'addiction comportementale est sélectionnée parmi le groupe constitué par une addiction à Internet, une addiction aux jeux, une addiction aux jeux d'argent, une addiction au sexe, une addiction aux achats, une addiction à l'exercice physique, et une addiction au travail.

3. Utilisation selon la revendication 1, dans laquelle l'anticorps est un anticorps polyclonal ou un anticorps monoclonal.

4. Utilisation selon la revendication 1, dans laquelle l'acide nucléique est une amorce ou une sonde.

5. Utilisation d'une trousse dans le diagnostic d'addictions comportementales, la trousse comprenant un agent capable de se lier spécifiquement à la protéine préséniline-1 ou un polynucléotide codant pour la protéine préséniline-1 afin de mesurer un niveau d'expression de la protéine préséniline-1 dans un échantillon biologique d'un sujet suspecté de présenter une addiction comportementale, dans laquelle l'agent est un anticorps ou un fragment de liaison aux antigènes de celui-ci se liant spécifiquement à la protéine préséniline-1 ; ou un acide nucléique comprenant un polynucléotide qui est identique ou complémentaire à un polynucléotide codant pour la protéine préséniline-1 ou un fragment de celui-ci.

6. Procédé de détection de la préséniline-1 pour le diagnostic d'addictions comportementales, le procédé comprenant :
la mesure d'un niveau d'expression de la protéine préséniline-1 dans un échantillon biologique qui est séparé d'un sujet suspecté de présenter une addiction comportementale ;
et la comparaison du niveau d'expression mesuré avec un niveau d'expression de la protéine préséniline-1 d'un groupe témoin normal.

7. Procédé selon la revendication 6, dans lequel l'échantillon biologique est du sang, du plasma, du sérum, du fluide de moelle osseuse, du fluide lymphatique, de la salive, du fluide lacrymal, du fluide muqueux, du fluide amniotique ou une combinaison de ceux-ci.

8. Procédé selon la revendication 6, dans lequel la mesure comprend l'incubation de l'échantillon biologique avec un anticorps se liant spécifiquement à la protéine préséniline-1.

9. Procédé selon la revendication 6, dans lequel la mesure est réalisée par électrophorèse, immuno-empreinte, dosage d'immuno-absorption par enzyme liée (ELISA), puces à protéine, immunoprécipitation, microréseau, transfert Northern, amplification en chaîne par polymérase (ACP), ou une combinaison de ceux-ci.
